# EUROPEAN PATENT APPLICATION

(11) **EP 4 356 820 A1**
(43) Date of publication of application: **24.04.2024**
(21) Application number: 22382996.1
(22) Date of filing: 18.10.2022
(51) Int. Cl.: A61B 5/00, A61B 5/107, G01B 11/25

(54) **METHOD AND SYSTEM FOR DETECTING AND OPTIONALLY MEASURING AT LEAST ONE DIMENSION OF ONE ORE MORE PROTUBERANCES OF AN AREA OF A SKIN SURFACE**

(71) Applicant: INNOPRICK, S.L., 20009 San Sebastián, Guipúzcoa (ES)
(72) Inventor: MATELLANES GARCÍA, Oscar, E-20014 San Sebastián, Guipúzcoa (ES); RICA REGUILÓN, Ane, E-20014 San Sebastián, Guipúzcoa (ES); TEJERO PASCUAL, Eduardo, E-20014 San Sebastián, Guipúzcoa (ES)
(74) Representative: ABG Intellectual Property Law, S.L.

(57) **Abstract**

The present invention is related to allergy detection and more specifically to methods and systems for detecting and optionally measuring at least one dimension of one or more protuberances, for example one or more papules, of an area of a skin surface that correspond to a positive reaction for detecting allergies.

## Description

### FIELD OF THE INVENTION

The present invention is related to allergy detection and more specifically to methods and systems for detecting and optionally measuring at least one dimension of one or more protuberances of an area of a skin surface, for example one or more papules that correspond to a positive reaction for detecting allergies.

### PRIOR ART

An allergy is a reaction of the human body when it comes in contact with a substance called allergen. This may provoke the appearance of a number of symptoms, such as rhinitis, itching, asthma, urticaria, etc., of varying severity, ranging from mild discomfort to severe health conditions that may even endanger a person's life.

Any sort of substance may cause an allergic reaction in people, by coming into contact with the patient in a variety of ways. For example, it may be a suspended substance found in the environment and breathed in by a person or patient, such as pollen or plant animal secretions, or through ingestion, as in the case of food or medicines.

The main remedy for an allergic person is to avoid contact with the allergen, especially in case of allergies which can be particularly severe. However, it is not always possible to completely isolate a patient from the allergen. Various treatments for allergies exist, following which the patient may stop showing symptoms to the allergen, thus allowing a normal life.

In order to be able to avoid contact with the allergen and in order to establish an effective treatment, an allergen must be clearly identified first. This is not always straightforward. To this cause, different methods have been developed for detection of allergies.

Typical methods for the detection of allergies involve subjecting the patient to a potential allergen under controlled conditions. Other known methods involve a blood analysis to validate or rule out an allergy.

The most commonly used method for detecting allergies is known as a "Prick Test". It consists of applying a small amount of allergen to be proved on the skin of a patient, usually on the inner forearm or on the back (in the case of children) of the patient, then making a micro-incision on the skin with a small knife, thereby bringing the allergen in contact with the body. As a consequence, after 10 to 15 minutes a small allergic reaction may take place that may form a protuberance with its center in the prick. This protuberance which appears in the skin of a patient is called wheal or papule.

Allergic papules usually have the shape of an ellipsoidal cap, which produces an ellipse-shaped area, although strange shapes sometimes occur (especially if the puncture is performed near a vein). The usual area of a papule is between 7 mm² and 200 mm², or what is the same, an average diameter between 3 mm and 16 mm.

The papules are used as the method to quantify a patient's sensitivity to an allergenic substance. In order to compare the different reactions that patients may present, these papules are parameterized, resulting in the maximum diameter of the papule, the minimum diameter of the papule, the average diameter, its area or its volume. In this way it is possible to compare one allergic reaction with another, either between the same patient on different days or between different patients. Therefore, the size and shape of the papule determine the degree of affliction to a certain applied allergenic substance.

In a next step, a doctor or nurse marks the contour of the papule with a pen or marker. This mark is then collected by providing a piece of adhesive tape on the mark so that the ink adheres to the tape. Then the piece of tape is placed on a graph paper, usually in an area of the paper that identifies the corresponding allergen. Then the number of squares of the maximum diameter of the papule is determined and written down. These results are then transferred to doctor, who determines a suitable prescription and adds it to the patient's record.

This "Prick Test" method is often applied simultaneously with different allergens, such that it is possible to simultaneously test multiple allergies.

The "skin Prick Test" method is relatively simple and fast, allowing the performance of various tests simultaneously. However, the analysis and measurement of the size of the papules that correspond to a positive reaction are performed in a totally manual and laborious manner, so that the "skin Prick Test" method lacks a certain degree of precision. Furthermore, for the prescription from the part of the doctor it is necessary to send the result on paper to the doctor's office, thus further lengthening and possibly delaying the process. It is also necessary to send the case history in order to properly review the result.

For all those reasons, in recent years major efforts have been focused on improving reading during allergic reactions, either by improving the "skin Prick Test" process or by completely changing the test methodology.

Document EP2967331 discloses a method of measuring one or more dimensions of one or more papules on a three-dimensional representation of an area of a skin of a forearm executed by a processor of a computer device. This is an automated method for determining dimensions of papules, which is much quicker and more reliable than prior art methods. However, this automated method does not solve the existing problem of properly identifying those papules that have a scale value greater than a value dependent on the cut-off values of the disclosed second filtering. Those papules that have a scale value greater than a value dependent on the cut-off values of the disclosed second filtering, which be referred as a "large papule", results in its interior not being considered as part of the structure of the papule. Due to this incorrect interpretation those "large papules" are not correctly identified according to the automated method described in document EP2967331, since the interior of these "large papules" is interpreted as a region with a configuration typical of the surface of the arm and not a perturbation, that is, part of the papule.

In reality, a filter affects height variations of a given scale and, therefore, filters gradients by eliminating those fluctuations that have a length scale below a certain value (high frequency scales in a Fourier approximation). In a papule, the high gradient values measured by means of a predetermined norm are mainly at the edge since, for "large" papules, the interior area is smooth and indistinguishable from any surface of the arm. For this reason, in the first method described in document EP2967331, the use of filters based on removing short-length scale fluctuations results in ring shaped protrusions representing papules when such papules are large and, therefore, the inner surface of the papules are not identified as being part of the papule.

Although the described prior art methods are based on the "skin Prick Test", the method and system of the present invention can also apply to other types of skin allergy tests such as intradermal allergy tests.

It would be highly desirable to find a method and a system for properly automated detecting and optionally measuring at least one dimension of one or more protuberances, for example one or more papules, and especially "large" papules, in a surface being modelled by means of a numerical model generated from data captured from a region of the skin of a patient.

### DESCRIPTION OF THE INVENTION

The present invention provides a solution for the above mentioned issues by a method of detecting and optionally measuring one or more dimensions of one or more protuberances, preferably one or more papules, of an area of a skin surface of a person or part of it according to independent claim 1, a measuring system of one or more protuberances, preferably one or more papules, of area surface of a skin surface of a person or part of it according to independent claim 13, a computer device according to independent claim 18 and a computer program according to independent claim 19. In dependent claims, preferred embodiments of the invention are defined.

*In a first inventive aspect, the present invention provides a computer-implemented method of detecting and optionally measuring at least one dimension of one or more protuberances, preferably one or more papules, of an area of a skin surface of a person or part of it, by means of a computer system; the method comprising the steps:*
*a) receive in the computer system at least a three-dimensional representation of a numerical model of an area scanned from the skin surface of a person and determine in the computer system a plurality of numerical first curved lines extended on the surface of the three-dimensional representation of the numerical model of an area of the skin surface, wherein the projection of each first curved line on a predetermined domain is oriented according to a predetermined first direction;*
*b) determine in the computer system, for each first curved line, a starting limit point and an end limit point of an area corresponding to a protuberance, preferably a papule, where the starting limit point and an end limit point correspond to a local maximum followed by a local minimum or, a local minimum followed by a local maximum respectively of the gradient along the second direction;*
*c) determine in the computer system the perimeter border of each protuberance, preferably each papule, in the three-dimensional representation by connecting the detected starting limit points and end limit points of an area corresponding to a protuberance, preferably a papule, of adjacent first curved lines; and*
*d) determine in the computer system at least one dimension of at least one protuberance, preferably at least one papule, responsive to the shape of the perimeter of said protuberance, preferably said papule.*

According to a further embodiment, the method further comprises the steps:
*e) add in the numerical model by means of the computer system an inner area delimited within each identified perimeter border of a protuberance, preferably a papule, from the three-dimensional representation by reconstructing it by interpolation from an adjacent region of the three-dimensional representation containing the protuberance, preferably the papule; and*
*f) determine in the computer system the space limited between the three-dimensional representation of said one or more protuberances, preferably one or more papules, and the inner interpolated area intended for reconstructing the surface with no protuberances, being the at least one dimension of one or more protuberances, preferably one or more papules (P1, P2), determined from the shape of said limited space.*

Preferably, the method and a system of the invention is focused on detecting and optionally measuring at least one dimension of one or more papules of an area of a skin surface of a person, wherein the one or more papule(s) is/are generated by a "skin Prick Test".

However, the present method and a system may be also applicable to detecting and optionally measuring at least one dimension of one or more protuberances of an area of a skin surface of a person, wherein the one or more protuberance(s) is/are not papules but other types of protuberances generated by other types of skin allergy tests such as intradermal allergy tests.

Hereinafter, the specific term "papule" will be used throughout the Description, although the method and system of the invention can also be applied for other types of papule-like protuberances, as mentioned in the previous paragraph.

Once the at least one dimension of one or more papules is determined in step "d", estimating the size of the detected papules on a three-dimensional representation of a numerical model of an area scanned from the skin surface of a person, e.g. the area or the volume, may then be performed according to specific embodiments either manually or automatically based on their three-dimensional representation.

Advantageously, this new method is capable of detecting and measuring with great precision and automatically one or more dimensions of one or more papules in a surface being modelled by means of a numerical model generated from data captured from a region of the skin of the patient. Therefore, it is a quicker and more reliable method in comparison with other known prior art methods. Besides, this new method is capable of detecting and measuring with great precision and automatically one or more dimensions of a "large" papule".

As mentioned above, the patient's forearm is usually used for skin testing. However, in certain cases, skin testing can be also performed on a patient's back, instead of the forearm. Possible examples where the back is used are: situations where it is easier to place allergens on the back than on the forearm, for example in the case of children where it is more difficult to immobilise the arm; or situations where a large amount of allergens have to be placed and there is no space on the forearm. Therefore, the present method is preferably intended to be used to detect and measure at least one or more dimensions of one or more papules on a skin surface of a forearm or back of a patient. However, this method is not limited to said two parts of the body, but can also be used for other parts of the skin surface of a person, such as the back.

In reference to the step "a" of the method, starting from a 3d representation of a numerical model of an area scanned from the skin surface of a person, e.g. a forearm, of a patient (i.e. the reconstruction surface), the computer system generates a plurality of numerical curved lines (called "first curved lines") extended on the surface of the 3d representation of the numerical model of an area of the skin surface. The projection of each first curved line on a predetermined domain is oriented according to a predetermined first direction.

Possible examples of 3d representations of a numerical model of an area scanned from the skin surface of a person are numerical meshes defined over a set of connected nodes representing points of the surface of the skin. As an example, some of the most common meshes are unstructured meshes comprising triangles or structured meshes such as those made by rectangles. The mesh is an example of numerical model representing a surface, wherein each point of the surface on the skin is approximated by the coordinates in the mesh or the specific numerical model representing the surface. Another example of numerical representation of a surface is a cloud of 3d points wherein each point is a point of the surface. In this case the management of geometric aspects of the surface can be more complex to calculate because the points are not necessarily connected.

In reference to the step "b" of the method, the first curved lines have a projection on the predetermined domain as a set of straight parallel lines, the set of parallel lines being also parallel to the first direction. For each first curved line of step "a", the computer system calculates the gradient along the first direction. This gradient is a wave shaped function and the computer system next searches for a pattern in the variation of this gradient wave. More specifically, the computer system searches for those regions of the gradient wave (called "RS" peaks region) that contain a positive wave (called "R" wave) followed by a negative wave (called "S" wave), or vice versa (a negative "S" wave followed by a positive "R" wave (for example, using a methodology based on searching pairs of local maximum followed by minimum peaks or pairs of local minimum followed by a maximum peaks that follow pattern of behaviour of a "RS"/"SR" region). Each of the detected "RS"/"SR" (herein after being referred to as "RS" for both cases) regions corresponds to a starting limit point and an end limit point of an area corresponding to a papule or vein that will be after eliminated to achieve a "correct" "base surface".

A "base surface" is defined as a reconstructed surface generated from the original scanned surface representing the surface of the skin with no papules. The "base surface" is generated according to the present method by removing a region of the original surface scanned identified as being associated to the papule from the skin and, replacing said removed region by an interpolated region using the remaining original region.

As a possible embodiment of the selection of these "RS" peaks region of step "b" of the method, the following actions are carried out by the computer system in this order:
b1) Optionally, the gradient wave is normalized by applying a filter to eliminate the highest frequency oscillations, which represents structures of very small scales such as hair.
b2) Pairs of positive and negative peaks having a certain predetermined difference of amplitudes between them, consisting of a positive peak (positive value) followed by a negative peak (negative value) are detected; or pairs of negative and positive peaks consisting of a negative peak (negative value) followed by a positive peak (positive value) are detected.
b3) Of these "RS" peaks region candidates to be detected, pairs of positive followed by negative peaks or pairs of negatives followed by positive peaks are eliminated and kept taking into account the difference of amplitudes of both peaks.

Once the final "RS" peaks regions have been detected in step "b" of the method, the computer system calculates the perimeter border of each papule in the 3d representation by connecting starting limit points and end limit points of adjacent second curved lines (step "c" of the method) determining a closed curve representing an inner region corresponding to the detected papule.

After that, an according to a specific embodiment, the portion of curve corresponding to the interval delimited by these final "RS" peaks positions is eliminated from the original curve (managing to eliminate papules, veins, etc. from the original signal).

Subsequently, the computer system adds an inner area delimited within each identified perimeter border of a papule from the 3d representation by reconstructing it by interpolation from an adjacent region of the 3d representation containing the papule (step "d" of the method). This interpolation is performed to obtain a "clean" "base surface" of the forearm.

Finally, also according to a specific embodiment, the computer system subtracts the original three-dimensional representation of said one or more papules from the "clean" "base surface" of the forearm (i.e. an inner interpolated area intended for reconstructing the surface with no papules), so resulting in a new numerical model corresponding to a limited space with the papules (step "e" of the method). The shape of this limited space is where the shape of one or more papules are determined and optionally being measured.

In a preferred embodiment of the invention, the method may also comprise a prior step of scanning of the three-dimensional area of the skin of the patient where the test is applied with a projected light beam and being reflected towards a camera comprising a two-dimensional sensor and also generating *in the computer system a numerical model comprising a three-dimensional representation (i.e.* reconstruction) *of an area of the skin surface.* Said scanning step may be performed either by the same apparatus as that generating the 3d representation of the papules or by a separate apparatus. This scanning and generation of the three-dimensional representation of the area of the skin requires the presence of the forearm -or other part- of the person. However, the generation of the 3d representation of the papules and the ensuing measuring of their size may be either performed just after the first scanning step, or at a later time since the second generation step of the three-dimensional representation of the papules does not require the presence of the person. Some prior art systems are based on using a scan or photo of the forearm before applying the allergen(s) and one after applying the allergen(s). On the contrary, with a method according to these embodiments, only a single scan or a scan in only one direction is required. In this preferred embodiment of the invention, *the previous method requires the use of the following means:*
- *a base intended for supporting the person or part of the person, the skin surface extended at least along a second direction when in operative manner;*
- *a light beam generator configured to emit a light beam to illuminate a line projected onto the skin surface along a second direction, and*
- *a first camera comprising a first sensor, the first camera being adapted to capture the projected line onto the skin surface and determinate its shape by generating an image of the skin surface when the person or part of it is rested on the base and; wherein the generated image also comprises a curve caused by the light beam when the skin surface is operatively illuminated by the light beam generator, the first camera at least comprising two optical parameters, the orientation of the focal axis and the location of the focal point; and*
- *a computer system in communication with the light beam generator and the first camera;*
*and the method, before step a), further comprises the steps 1), 2) and 3):*
*step 1) capture a plurality of images of an area of the skin surface of the person or part of it when, in operative manner, the person or part of it is resting on the base, the plurality of images taken by the first camera while illuminating the area of the skin surface by the light beam generator and, each image taken while illuminating a line at a different location of the area of the skin surface;*
*step 2) receive in the computer system from the first camera the plurality of images and determine a plurality of numerical second curved lines in a space, at least one curved line for each image of the plurality of images, each second curved line in space determined by calculating the spatial position of a set of pixels of the illuminated line appearing in the image and responsive to the optical parameters of the first camera; and*
*step 3) generate in the computer system a numerical model comprising a three-dimensional representation of an area of the skin surface, the model determined from the plurality of second curved lines in space.*

The following terminology is used along the whole document to describe features of the invention:
- The term "large papule", as used in this application, shall refer to a papule that have a length scale value greater than a value dependent on the cut-off length scale value of the disclosed second filtering.
- The term "three-dimensional representation" or its abbreviation "3d representation", as used in this application, shall refer to a digital representation of the area of the skin of a patient wherein each point of the surface is determined by three coordinates.
- The term "a dimension of one or more papules", as used in this application, shall refer to a dimension, such as maximum diameter, orthogonal, height, area and volume.
- The term "an area of a skin surface" of a patient, as used in this application, shall refer to, for example, an area of the skin of a forearm of a patient. However, other parts of a patient may be susceptible to be measured by this method and system.
- The term "camera", as used in this application, shall refer to a camera with at least one two-dimensional sensor for capturing an image, and an optical component that allows an image to be projected onto the sensor according to certain intrinsic parameters such as the direction of an optical axis or the focal length.

It is precisely the optics of the camera and the knowledge of its intrinsic parameters as well as the position and orientation of the camera sensor in space that allows that from a captured image, each of the pixels can be made to correspond to a point in space that has given rise to the pixel in the image.

In a specific embodiment the position of the point of the surface in space is determined using two lines.

The image is considered to be captured by a sensor of the camera located and oriented in space according to known parameters. Thus each pixel of the image corresponds to an element of the observed space illuminated by a light beam (for instance a laser or a LED beam) which results in the degree of illumination of a pixel of the sensor. From this construction, a first line fulfils to comprise the point of the observed space, the location of the pixel and the focal point of the camera. In this case the element of the observed space is the surface of the patient's skin that has given rise in particular to the pixel being illuminated in the image.

The complete image is generated by scanning the light beam along a line. The result is an image with illuminated pixels that determine a curve.

Thus, departing from the image generated, specifically from a pixel of the curve drawn by illuminated pixels, a line passing through the pixel and the focal point is defined. This line is the first line.

The second line is the one that represents the trajectory of the light beam impinging on the point in the observed space. This is the second line.

If the precision of all the elements were infinite, the determination of the coordinates of the point in space of the observed element can be obtained as the intersection of the first line and the second line. However, as soon as there is some kind of inaccuracy, both lines cross and do not intersect. Thus, the point closest to one and the other curve is considered to be the searched point. In the particular case that both curves intersect, the intersection point is still the closest point to both curves. Searched point is the point determined by a pixel of the curve now located in space.

In a further embodiment the position of the point of the surface in space is determined using a line and a plane. This is the example where instead of a light beam there is a light emitter that illuminates a line contained in a plane.

In this embodiment the first line is determined from the pixel position and the camera parameters as described in the previous example.

However, now the position of the searched point is determined by calculating the intersection between the first line and the illumination plane.

Steps "a" to "e" do not require the presence of the patient. Therefore, they may be performed simultaneously or at a later time than steps "1 to 3" of capturing of a plurality of images of an area of the skin surface of the person or part of it with one or more papules by a first camera while illuminating the area of the skin surface by a light beam generator and generating in a computer system a numerical model comprising a three-dimensional representation of an area of the skin surface.

According to steps "1 to 3" of this preferred capturing steps, the capturing process is performed only once over the part of the person after applying the allergen(s); therefore, it is very quick process since it is not necessary to scan or photo the part of the person before applying the allergen(s) and one after applying the allergen(s) (which was necessary by some prior art systems).

In an embodiment, *the person (also called patient) or part of it is resting along the second direction and the focal axis of the first camera is transverse to the second direction.* In this particular embodiment, the light beam projected onto the skin may correspond to a line projected onto the skin surface, preferably a line which is contained by a plane parallel to the second direction. By emitting a line on the area of a skin surface of a person of part of it and receiving its reflection at a sensor of the first camera, it is possible to obtain a set of pixels (illuminated pixels) for which it is possible to determine their position in space. The connection of the points in space determined from the pixels of the illuminated curve gives rise to a curve in space that makes it possible to define part of the numerical model representing the skin surface. A plurality of sections of the area of a skin surface of a person of part of it, e.g. an area of a forearm, may thus be obtained. This represents an efficient manner of obtaining a cloud of points arranged in curves almost parallel for a portion of e.g. a forearm. In this particular embodiment, the first part of method step "1" corresponding to the capture by the first camera of the plurality of images taken from the area of the skin surface of the person or part of it with one or more papules being illuminated by a light beam may comprise displacing a light beam generator generated by a light beam generator in a direction transverse to the second direction (in case of being a forearm), emitting said light beam.

According to a first embodiment of the invention, the light beam generator is configured for being displaced according to the transverse direction to the second direction to emit the light beam according to a displaced and parallel plane.

According to a second embodiment of the invention, the light beam generator is configured to emit a light beam according to a plane that is changing in inclination to sweep the scanned area according to the direction transverse to the second direction so that it is not necessary to move the light beam generator.

According to a third embodiment of the invention, the light beam generator is configured for emitting a light beam according to a combination of the first and second embodiment, i.e. the light beam generator is configured for being displaced according to the transverse direction to the second direction to emit the light beam according to a displaced and parallel plane and it is also configured for emitting a light beam according to a plane that is changing in inclination to sweep the scanned area according to the direction transverse to the second direction so that it is not necessary to move the light beam generator.

The set of curves determined after scanning the area of a skin surface determines a numerical model representing this surface. According to a preferred embodiment, the numerical model is generated applying a three-dimensional triangulation algorithm to generate a mesh, the three-dimensional representation of the area of the skin surface with one or more papules. Other meshes can be used for generating the numerical model comprising a surface representation such as structured meshes or unstructured meshes where the connection between nodes is not necessarily by means of triangles.

As the light beam generator is displaced along a direction transverse to the second direction, the light beam may quickly sweep the area where the one or more papules are present. The reflection of said light beam may be recorded by a receiving sensor of the first camera, so according to a preferred embodiment generating a record of the elevation of the skin at distinct points by applying a suitable triangulation algorithm allows a numerical model representing the scanned surface of the skin. A grid or cloud of points representing a three-dimensional representation of a curve of the skin may also be obtained. As it has been disclosed, a cloud of points is an alternative manner of determining a numerical model representing the surface of the skin.

The laser triangulation system has some advantages over the other possible scanning systems, such as:
- Understanding the internal software is easier.
- Scan resolutions are more versatile and easier to change.
- The number of data to be transferred between the laser system and the computer system is less, achieving shorter transmission times.
- The data output is appropriate for the subsequent use that will be given to it by means of mathematical programs.

In some embodiments, the scanning of the area of the skin with a light beam to generate said three-dimensional representation of the area of the skin may comprise displacing a light beam generator in a direction, preferably transverse to the axis of the forearm, emitting said light beam along a direction parallel to the axis of the forearm, and subsequently applying a three-dimensional triangulation algorithm to generate said three-dimensional representation of the area of the skin.

According to other embodiments, once the first scanning is done, a sequent scanning is done in an adjacent area of the skin surface, preferably displacing the light beam generator in a direction transverse to the axis of the forearm (or transverse to the second direction) to another position, so that the light beam emits onto another adjacent area along a direction parallel to the axis of the forearm until the light beam may sweep the whole area where the one or more papules are present. By emitting the light beam in a direction parallel to the axis of the forearm and displacing the light beam generator transverse to the axis of the forearm, the time required for scanning the area of the forearm is reduced in comparison of scanning in the transverse direction while moving or sweeping along the longitudinal direction (the standard measurement of a forearm of a 95% European male percentile is 300 mm long and 100-120 mm in diameter at its widest part), which substantially reduces the time spent on scanning the whole area of the forearm where the one or more papules are present.

In some embodiments, displacing the light beam generator may comprise a controlled speed displacement. This allows associating each light beam with the position on the skin where it is reflected. In other embodiments said displacing may comprises an arbitrary speed displacement. It is then beneficial to register the position of the light beam (e.g. using a suitable encoder) for each section of the forearm in order to associate each light beam with the position on the skin.

In a preferred embodiment, *the light beam generator may* comprise a light source, *preferably a laser or even a very low power light source such as a LED and a suitable lens system.* As an example, the laser may be a laser with a wavelength of 633 nm and a maximum power of 50 mW.

In an embodiment, *the predetermined first direction may be the second direction.*

In an embodiment, *the predetermined domain is parallel to the base and the projection of the first curved lines in the predetermined domain are straight parallel lines and, being equispaced.*

According to these conditions, the projection of the first set of curves greatly facilitates the subsequent calculation of gradients since the curve is projected directly according to a coordinate direction along which it is possible to calculate derivatives and an individualized curve-by-curve treatment.

In an embodiment, *the pair of starting limit point and end limit point may be discarded if they are separated in the first direction more than a first predetermined length value.*

The application of this criterion makes it possible to discard points that would give rise to small papules that are identifiable by other more efficient and more precise means. It also eliminates the identification of regions with fluctuations due to small-scale disturbances that generate noise.

In an alternative embodiment, *the pair of starting limit point and end limit point may be discarded if they are separated in a direction transversal to the first direction more than a second predetermined length value.*

The shape of a papule will preferably be defined by several first curves. This criterion makes it possible to eliminate the link between two sufficiently distant curves that would otherwise result in the connection of two independent papules.

In a second embodiment of the invention, *the method further comprises:*
- *a second camera comprising a second sensor, wherein the second camera is adapted to generate an image of the skin surface of a person or part of it when is rested on the base and the curve caused by the light beam when operatively illuminated by the light beam generator, the second camera at least comprising two optical parameters, the orientation of the focal axis and the location of the focal point;*
- *a synchronization module between the first and the second camera to obtain pairs of images at the same instant of time; and*
*the computer system being in communication with the light beam generator, the first and second cameras and the synchronization module, wherein in step a) the computer system is further configured to:*
*i. command the activation of the light beam generator illuminating the skin surface with the light beam capturing a first image with the first camera and a second image with the second camera along a plurality of sections of the skin surface extended at least along the first direction, each pair of the first image and the second image comprising pixels corresponding to the same illuminated line on the skin;*
*ii. for each pair of the first image and the second image, determining a plurality of points in the space satisfying that each point corresponds to the intersection of a first straight line connecting an illuminated pixel of the first image and the location of the focal point of the first camera and, a second straight line connecting an illuminated pixel of the second image* of the *same location on the skin surface and the location of the focal point of the second camera; and*
*iii. generating the curved line of step 2) by connecting the set of points determined in former step ii).*

According to this second embodiment, each of the curves is formed from points determined not only by one pixel but by two pixels, one pixel of each image captured simultaneously on the same line illuminated by the laser beam. This second image enriches the information on the exact position of the line illuminated by the laser beam. In particular, when an image allows determining a point in space, it is because it has sufficient ability to focus that point along the line connecting the focal point of the board and the illuminated pixel in the sensor image once the focal point of the board and the pixels of the sensor are positioned in space. However, the depth of field of the optical system of the camera establishes a threshold of uncertainty in the direction of the line connecting the focal point and the pixel in the image, both located in space.

The second image, captured from a different position and also with a different orientation direction, establishes a new line in space connecting the focal point of the second camera and the pixel corresponding to the same illuminated point. The two lines either intersect at the point in space corresponding to the illuminated point or uniquely determine a point considering the condition of minimum distance to each other.

It has been proved that according to this embodiment, the curve determined in space according to this embodiment has a much higher degree of accuracy.

In a possible embodiment of the method, *further a lens distortion of the first camera, a lens distortion of the second camera or both are corrected by the computer system after capturing the image.*

According to this embodiment, a prior aberration or lens distortion correction allows subsequent calculations based on the connection of points located in space to result in more precise straight lines and therefore in a point-to-point positioning of all the points defining the curve, especially in those regions of the image far from the optical center and more prone to aberrations.

Optionally, *the computer system it further adapted to execute a sub-step of applying a first filtering stage applied to the three-dimensional representation after being determined from the plurality of second curved lines in space or applied to the 2D images, wherein said first filtering stage is configured to filter out any irregularities of the three-dimensional representation or to the 2D images below a first predetermined length scale, so generating a first pre-filtered three-dimensional representation to be used in step 2).*

The capture of points by scanning a skin surface gives rise to certain fluctuations that may result in noise signals in the subsequent results. The sources of noise can be diverse such as the lack of precision of each of the elements involved in the capture such as the finite resolution of the image acquired by the image sensor of the camera. The application of this first filter cleans the signal obtained.

Additionally, *the computer system it further adapted to execute a sub-step of applying a second filtering stage to the first pre-filtered three-dimensional representation, wherein said second filtering stage is configured to further filter out any irregularities of the first pre-filtered three-dimensional representation below a second predetermined size and over the first predetermined length scale, so generating a second pre-filtered three-dimensional representation to be used in step 2).*

Even a clean signal due to the use of the first filter may be representing entities with a small spatial scale with respect to the papules and not providing information. This is the case of small particles that may be present on the skin surface or especially due to the presence of hair. This second filter makes it possible to determine a skin surface that does not represent these structures and only takes into account the shape of the papules.

In a second inventive aspect, the present invention also provides a detecting and optionally measuring system of one or more papules of area surface of a skin surface of a person or part of it. *The detecting and optionally measuring system* of one or more papules may comprise a device and a computer system. The device comprises at least a base, a light beam generator and a first camera, and the computer system is in communication with the light beam generator and the first camera. *The base is configured for supporting the person or part of it, the skin surface extended at least along in the second direction when the system is in operative manner.* The *light beam generator is configured in such a way as to emit a light beam to illuminate a line projected onto the skin surface along the second direction.* The *first camera comprises a first sensor, wherein the camera is adapted to receive a reflection from the light beam emitted on the forearm and generate an image of the skin surface when the person or part of it is rested on the base and, wherein the image also comprises a curve caused by the light beam when the skin surface is operatively illuminated by the light beam generator, and wherein the first camera at least comprises two optical parameters: the orientation of the focal axis and the location of the focal point. The computer system is being adapted to:*
*command the first camera causing to capture a plurality of images of an area of a skin surface of a person or part of it resting on the base, the plurality of images taken by the first camera while illuminating the area of the skin surface by the light beam generator and, each image taken while illuminating a line at a different location of the area of the skin surface;*
*receive from at least the first camera the plurality of images and to determine a plurality of numerical curved lines in a space, identified as second curved lines, at least one curved line for each image of the plurality of images, each second curved line in space determined by calculating the spatial position of a set of pixels of the illuminated line appearing in the image and responsive to the optical parameters of the first camera, in particular the focal axis and the location of the focal point;*
*generate a numerical model comprising a three-dimensional representation of an area scanned from the skin surface of a person or part of it, the model determined from the plurality of second curved lines in space;*
*determine a plurality of numerical curved lines, identified as first curved lines, extended on the surface of the three-dimensional representation of the numerical model of an area of the skin surface of a person, wherein the projection of each first curved line on a predetermined domain is oriented according to the predetermined second direction;*
*determine, for each first curved line, a starting limit point and an end limit point of an area corresponding to a papule (P1, P2), where the starting limit point and an end limit point correspond to a local maximum followed by a local minimum or, a minimum followed by a local maximum respectively of the gradient along the first direction;*
*determine the perimeter border of each papule in the three-dimensional representation by connecting the detected starting limit points and end limit points of an area corresponding to a papule of adjacent first curved lines;*
*determine at least one dimension of at least one papule responsive to the shape of the perimeter said papule;*
*making the at least one dimension available for instance by means of an output communication line or a display.*

The computer system is further adapted to:
*add in the numerical model an inner area delimited within each identified perimeter border of a papule from the three-dimensional representation by reconstructing it by interpolation from an adjacent region of the three-dimensional representation containing the papule; and*
*determine the space limited between the three-dimensional representation of said one or more papules and the inner interpolated area intended for reconstructing the surface with no papules, being the shape of the papule determined by the shape of said limited space.*

The base may be configured either by a table or a support where the rest of the device is installed, or a base *per se* integrated with the other components of the measuring system, such as the light beam and the first camera. The base may be ergonomically adjusted to allow the person to rest their arm on the base for an amount of time that may be required for the two-dimensional sensor to generate the three-dimensional representation of the forearm.

In a possible embodiment, *the measuring system further comprises:*
- *a second camera comprising a second sensor, wherein the second camera is adapted to generate an image of the skin surface of a person or part of it when is rested on the base and, the curve caused by the light beam when operatively illuminated by the light beam generator, the second camera at least being characterized by two optical parameters, the orientation of the focal axis and the location of the focal point; and*
- *a synchronization module between the first camera and the second camera configured to obtain pairs of images at the same instant of time; and*
*the computer system being in communication with the light beam generator, the first and second cameras and the synchronization module, wherein the computer system is further configured to:*
*i. command the activation of the light beam generator illuminating the skin surface with the light beam, an sending a signal to the first and second camera capturing a first image with the first camera and a second image with the second camera along a plurality of sections of the skin surface extended along the first direction, each pair of the first image and the second image comprising pixels corresponding to the same illuminated line on the skin surface;*
*ii. for each pair of the first image and the second image, determining a plurality of points in the space satisfying that each point corresponds to the intersection of a first straight line connecting an illuminated pixel of the first image and the location of the focal point of the first camera and, a second straight line connecting an illuminated pixel of the second image of the same location on the skin surface and the location of the focal point of the second camera; and*
*iii. generate the curved line by connecting the set of points determined in former step ii).*

The advantages of having a second camera are previously described in the method section and may apply for the system and are not repeated.

The at least one camera will have the necessary range and resolution requirements.

The at least one camera is located in the device displaced as well as inclined with respect to the laser beam generator (which occupies a central position). In this position the at least one camera is able to capture the relief that the reflection of the laser beam transmits. Once the projection of the light beam in the form of a line is projected on the surface to be scanned, the at least one camera sensor is able to see said line, which will no longer be straight, but will have taken the shape of the profile as it is viewed from an angle.

In a possible embodiment when two lateral cameras are provided, the first and second cameras are located in a respective fixed position at respective lateral positions in respect to the position intended for the skin surface and wherein the first and second cameras are preferably angled at 90º to each other.

In a possible embodiment, the at least the first and/or the second camera of the measuring system are monochrome cameras.

In a possible embodiment, the first camera further comprises a wavelength filter of the laser to avoid the influence of light in other wavelengths and be able to only see the laser line in the image.

In a possible embodiment, *the light beam generator is configured to project a light beam parallel to the second direction and the light beam generator is displaceable along a first direction, perpendicular to the second direction and parallel to the base.* In some embodiments, the light beam emitted onto the forearm may correspond to a line projected onto the skin such that a two-dimensional representation of a plurality of sections of the forearm is obtained. The plurality of said two-dimensional representations of sections of said at least one portion of the forearm may form the basis to generate the three-dimensional representation of the area with the use of a triangulation algorithm.

In a possible embodiment, the system may further comprise a frame. The frame may carry the light beam generator in a central position and the at least one camera. In one implementation, the frame may comprise two legs placed vertically which are connected by a crossbar. Each guide may be placed diametrically opposed to the forearm. The other end of the legs may be attached to a transverse member. The transverse member may comprise the light beam generator and/or camera. In an embodiment where there is a second camera, the first and second cameras are disposed at respective lateral positions of the crossbar.

In a possible embodiment, the system may further comprise at least one guide, substantially transverse to the second direction. The light beam generator may be movable along said at least one guide. The guide allows the direction that the light beam generator and corresponding sensor move and scan the forearm to remain substantially constant so that the two-dimensional sections generated as it scans the forearm do not intersect. For example, the guide may be in the form of a rail and the light beam generator may comprise wheels adapted to be coupled to the rail. One skilled in the art may appreciate that any type of guiding system suitable for guiding the light beam generator transverse to the first direction may be used. According to a further embodiment, the light beam generator is adapted to emit a light beam according to a plane that is changing in inclination to sweep the scanned area according to the direction transverse to the second direction so that it is not necessary to move the light beam generator. In this case, the plurality of planes containing the light beam emitted for each scanned line has a shape resembling the pages of a book turned upside down.

In a possible embodiment, the system may further comprise a motor to displace the light beam generator along said first direction transverse to the second direction. The motor may cause the light beam generator to hover in the first direction in a steady speed, thus allowing an even scanning of the forearm where each two-dimensional section will be substantially at equal distance with any previous or subsequent section. In some implementations the motor may have each position of the light beam generator and/or sensor registered with a reference system.

However, even if no steady speed is accomplished, it may still be possible to register two-dimensional sections. In a possible embodiment, the system does not include a motor to displace the light beam generator along said second direction transverse to the first direction. In this case where no motor is provided, the laser beam is movable through a scanner (set of mirrors and optics) that changes the direction of emission so that the beam contained in a plane changes plane. That is, the plane that contains the laser beam remains parallel to the first direction but is tilted to sweep the arm.

Any of the embodiments of the system and method may optionally include a filter with the wavelength of the light emitted by the light beam generator causing that the captured images only show the illuminated parts of the skin wherein the rest of the image does not show any light.

According to this new method and system, several advantages are obtained:
- Automating the "Prick Test" process, in such a way that a device may read each and every one of the allergic reactions presented by the patient, thereby leaping from the qualitative values obtained today to quantitative values that allow better diagnosis by the allergist.
- Increasing the current measurement precision, evolving the "Prick Test" methodology according to the current technology present in hospitals, instantly obtaining parameters such as the maximum diameter, the average diameter and the area, in addition to avoiding possible errors associated with a manual process (incorrect reading, confusion in assigning the allergen, misplacing documents...).
- Reducing the time consumption of health personnel currently dedicated to the practice of the "Prick Test", as well as reducing the diagnosis time.
- Creating a simple user interface that allows reading, analyzing and visualizing, allowing the allergist to generate a database for the diagnosis and clinical history of the patient, taking advantage of obtaining quantitative parameters and allowing the results to be extrapolated to other cases regardless of the hospital where the tests were performed.
- Accurately scanning, locating and measuring "large papules" generated by the Prick Test on a skin surface of a patient.

In a possible embodiment, the system may further comprise a computing module, configured to associate the three-dimensional representation of the one or more papules with one or more applied allergens and generate a measurement report of said three-dimensional representation. The report may include the size of the papules or may include information that may allow a practitioner to derive the size of the papule, such as three dimensional views or representations of the papules in a pre-sized matrix. The measurement report may, for example, comprise an indication of at least the height or the volume of said one or more papules. The size of the papule may be an indication of the reaction level of the patient to the allergen.

According to another embodiment, the system comprises a third RGB camera located in the upper part. This third RGB camera is configured for obtaining a color image of the skin surface, allowing to over-impose the identification of papules over the real image or as part of an automatic report of the patient.

In a third inventive aspect, the present invention provides *a computing device.* The computing device may comprise *a* memory, e.g. a non-transitory memory, *and a processor. The memory may store computer program instructions executable by the processor. Said instructions* may comprise *functionality to execute a method of detecting one or more papules of an area of the skin surface of a person or part of it according to steps a) to e) and optionally steps 1), 2) and 3) of the method* disclosed herein.

In a fourth inventive aspect, the present invention provides *a computer program product.* The computer program product may *comprise instructions which, when the program is executed by a computer system, cause the computer system to perform the steps a) to e) and optionally steps 1), 2) and 3) of the method* disclosed herein. The computer program product may be stored in recording media or carried by a carrier signal.

### DESCRIPTION OF THE DRAWINGS

These and other features and advantages of the invention will be seen more clearly from the following detailed description of a preferred embodiment provided only by way of illustrative and non-limiting example in reference to the attached drawings.

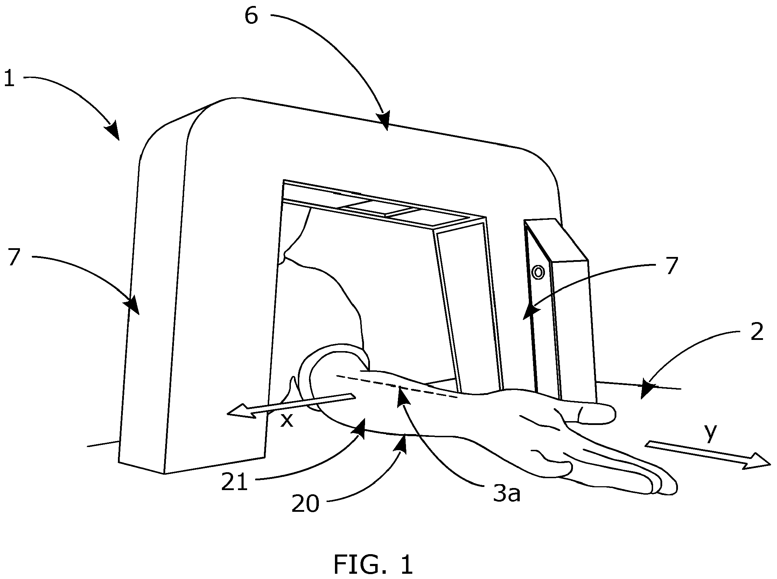

Figure 1 This figure shows a perspective view of an example of a device for measuring papules, including a patient with its forearm positioned inside the device.

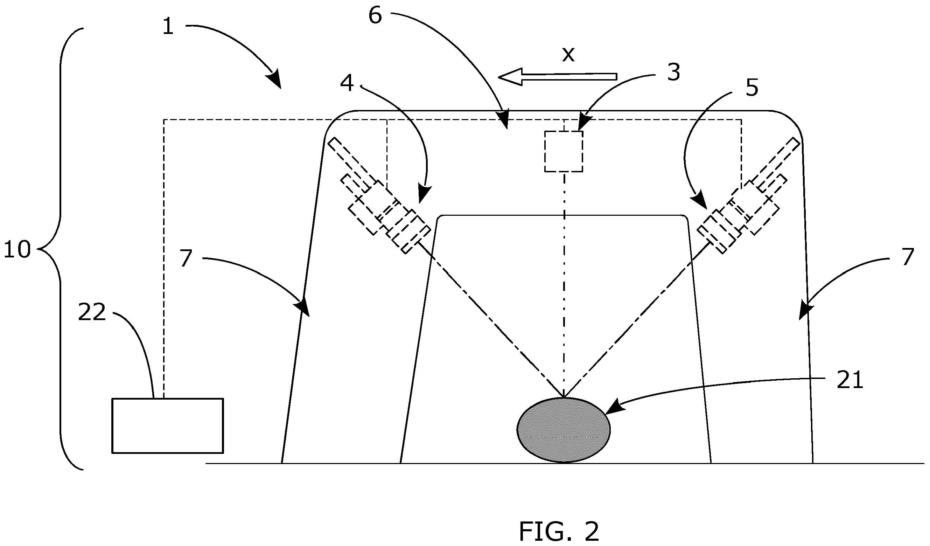

Figure 2 This figure shows a front view of a schematic representation of the example of a device for measuring papules of figure 1 and a computer system, including a patient's forearm positioned inside the device.

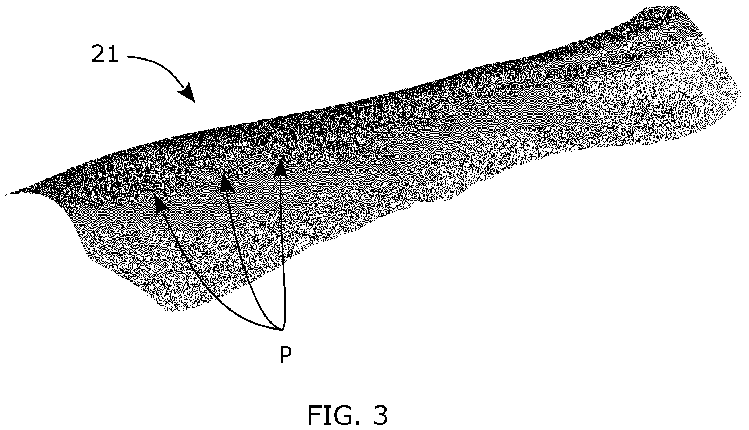

Figure 3 This figure shows a perspective view of part of a forearm exterior surface of a patient with three papules, once a "Prick Test" is applied for detecting allergies in the patient.

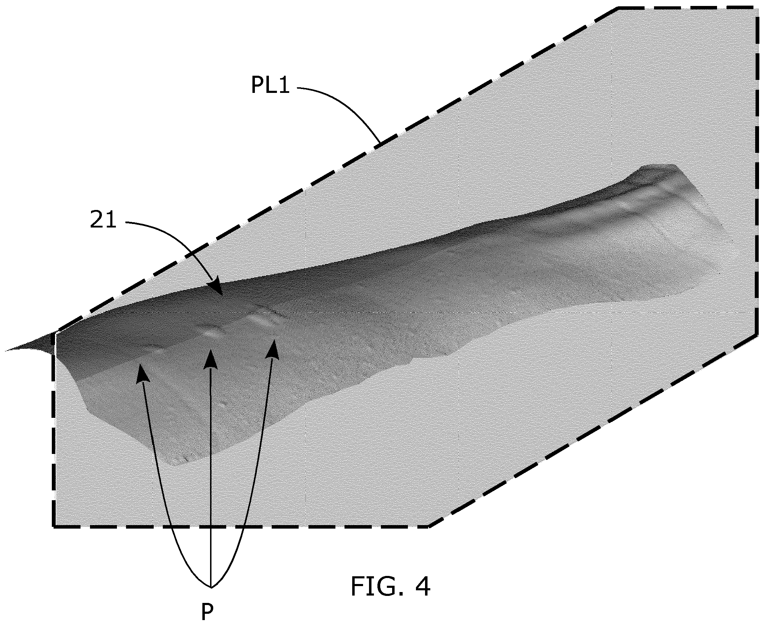

Figure 4 This figure shows the same perspective view as figure 3 of an example of a forearm of a patient, above which a certain plane containing the images with the curve positioned in space x-y-z is also represented.

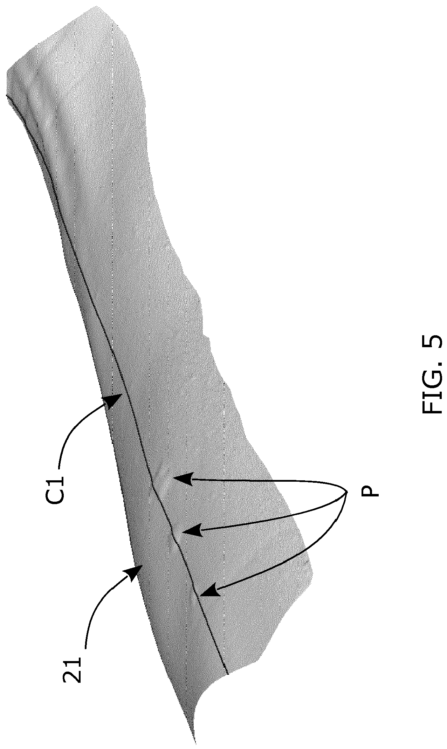

Figure 5 This figure shows the same perspective view as figures 3 and 4 of an example of a forearm of a patient, above which a certain plane (not shown) containing the reflected images with a curve positioned in space x-y-z is also represented for generating a numerical model of the surface.

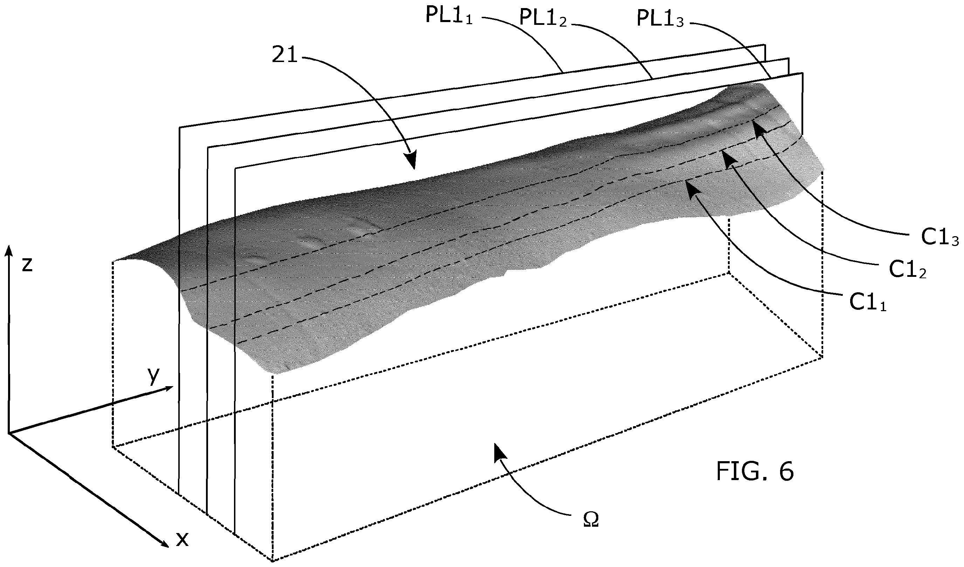

Figure 6 This figure shows the same perspective view as figures 3 to 5 an example of a forearm of a patient, above which different planes are represented, wherein each plane contains the respective reflected image according to a curve positioned in space x-y-z. These different planes can be either parallel between them or having another configuration, such as the pages of an open book. From the different curves of each plane positioned in space a three-dimensional representation of a generated numerical model of an area of the skin surface of a person is generated in a computer system.

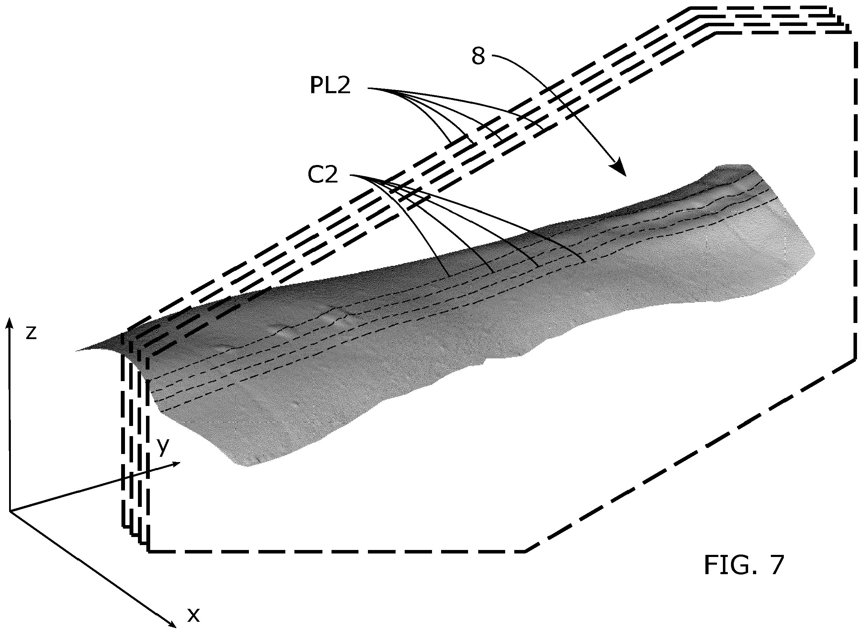

Figure 7 This figure shows the step "a" of the method, where an example of a 3d representation of a generated numerical model of an area of the skin surface of a forearm of a patient (i.e. the reconstruction surface), along with a plurality of numerical curved lines, called "first curved lines", extended on the surface of the 3d representation of the generated numerical model are represented.

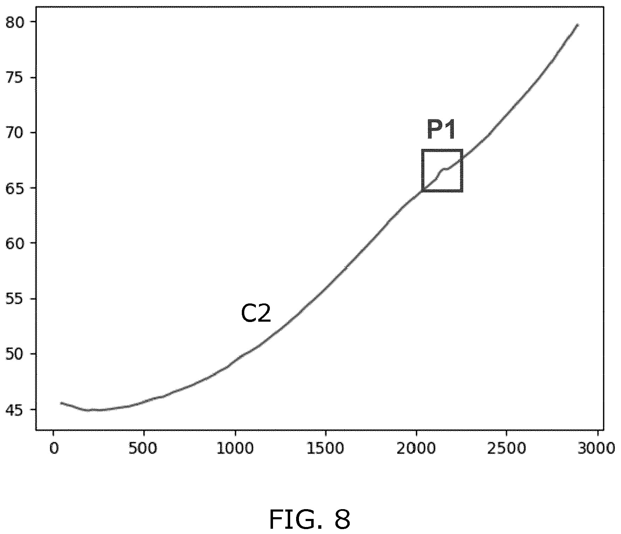

Figure 8 This figure shows a graphic where a specific vertical cut (of step "a") of the 3d representation of a generated numerical model of an area of the skin surface of a forearm of a patient containing a papule is represented, wherein this vertical cut, vertical in respect to the domain, generates a "first curved line" extended along the second direction, and the position of the papule in this "first curved line" is highlighted by a square.

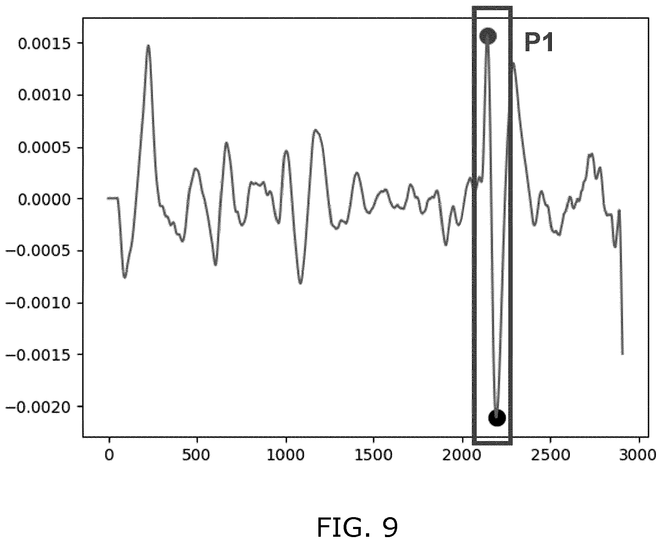

Figure 9 This figure shows the step "b" of the method, where the gradient of the specific first curved line of the vertical cut of figure 8 is calculated in the computer system and represented, and after that "RS" peaks regions are searched corresponding to a papule or vein that will be after removed to achieve a correct base surface.

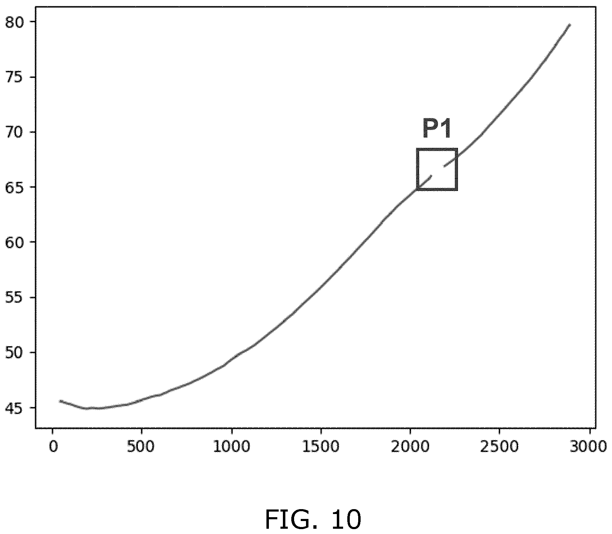

Figure 10 This figure shows a step of an embodiment of the method, where the original signal (i.e. a first curved line of the 3d representation of a generated numerical model of an area of the skin surface of a forearm of a patient) from which the part corresponding to the papule (i.e. the detected RS" peaks regions in step "b") has been eliminated, so managing to eliminate papules and/or veins, etc. from the original signal.

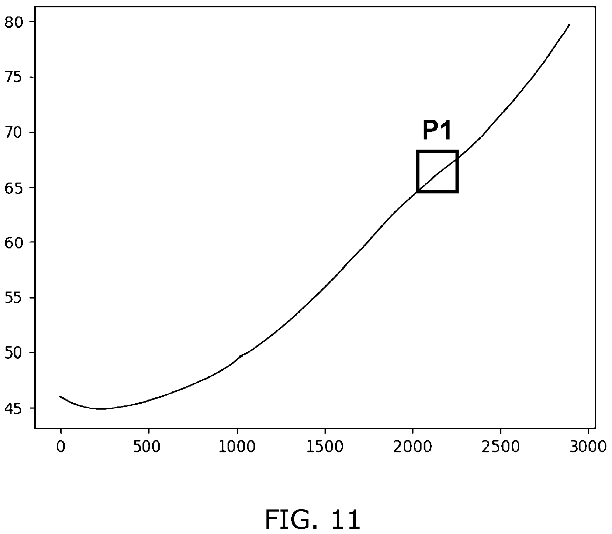

Figure 11 This figure shows the step "e" of the method, where an interpolation is performed to obtain a "clean" base surface of the skin surface of a forearm of a patient. This figure 11 depicts a cut interpolated signal, which would represent the base of the skin surface of a forearm of a patient.

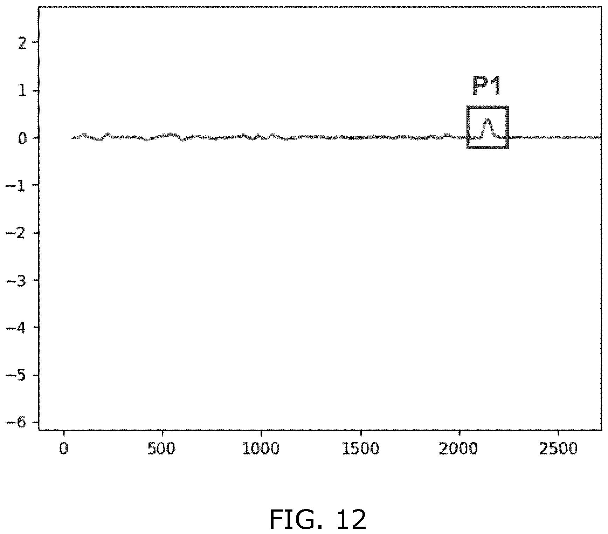

Figure 12 This figure shows the step "f" of the method, where a final new signal, after subtracting the original signal of the 3d representation of said one or more papules from the base curve of previous step, is depicted. This final new signal is where the papules are searched and at least one dimension of the papule/s is measured.

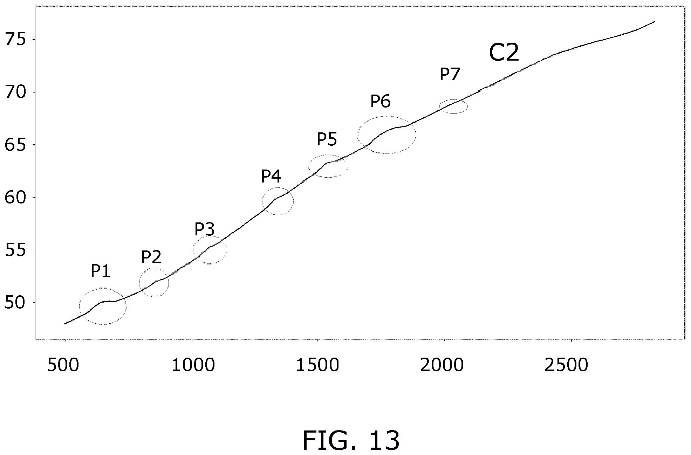

Figure 13 This figure shows the same graphic as figure 8 but according to a second example, where a specific longitudinal cut (of step "a") of a 3d representation of a generated numerical model of an area of the skin surface of a forearm of a patient is represented.

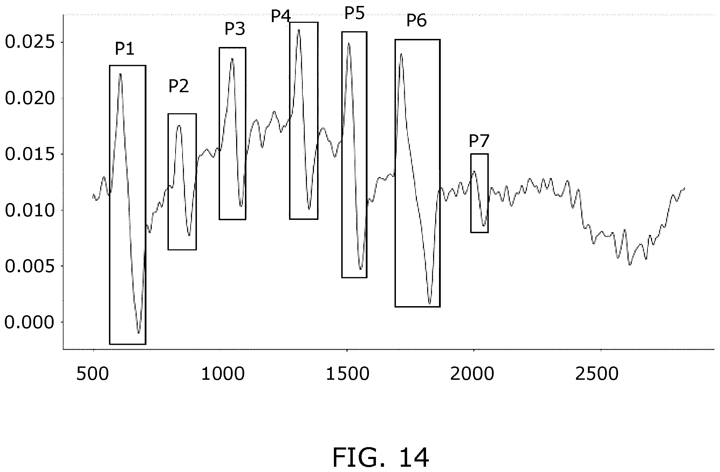

Figure 14 This figure shows the same graphic as figure 9 but according to the second example shown in figure 13, where the gradient of the specific first curved line of the vertical cut of figure 13 is calculated in the computer system and represented in this graph. The computer system also searches the "RS" peaks regions corresponding to those papules or veins that will be after eliminated to achieve a correct base surface.

### DETAILED DESCRIPTION OF THE INVENTION

As will be appreciated by one skilled in the art, aspects of the present invention may be embodied as a system, method or computer program product.

The present disclosure provides a preferred example of a method and system 10 for detecting and optionally measuring papules for the main purpose of detecting and parameterization of allergies in a patient. They allow analysis and processing of the results obtained by an automated or semi-automated "Prick Test" for allergies. The proposed methods are partly based on the conventional method for detecting allergies known as a "Prick Test". However, instead of manually registering on paper an edge of a papule, papules may be digitized and integrated into a software application that in some examples may record, analyze and electronically archive the results.

The detecting system 10 of one or more papules P1, P2 of area surface of a skin surface 20 of a person or part of it comprises a device 1 and a computer system (22). The device 1 comprises a base 2, a light beam generator 3 and a first camera 4. The computer system (22) is at least in communication with the light beam generator 3 and the first camera 4.

Figures 1 and 2 illustrates perspective and front views, respectively, of a device 1 for detecting papules P1, P2, according to an example. Both figures show the device 1 during operation, where the patient's forearm 21 is positioned inside the device 1. When the forearm 21 is substantially motionless, a user of the system 10, such as hospital personnel, a medical practitioner or a nurse, may turn on the system so that the scanning operation may begin. In both figures the papules (P1, P2) are not represented, but should be present in the exterior skin surface 20 of the forearm 21 of the patient. Moreover, figure 2 also represents the computer system (22) being connected to the light beam generator 3 and the first camera 4 by a dotted line.

According to this example, the device 1 may comprise a base 2 for supporting a part of the body of a patient, for example a forearm 21, and also for supporting the structure of the device 1. However, the structure of the device 1 may be configured to receive other parts of the body of a patient, such as the back. As shown in figure 1, the forearm 21 of a patient is in operative manner disposed extended at least along in a second direction corresponding to axis "y".

The structure of the device 1 may comprise a transverse head part 6 supported by a pair of columns 7 that are supported on the base 2. In some embodiments, the transverse head part 6 may be integrated to the pair of columns 7, so being a solid body. The structure of the device 1 according to this embodiment does not move in respect to the base 22.

The transverse head part 6 may incorporate a sensor having a light beam generator 3 configured to emit a light beam 3a to illuminate a line projected onto the skin surface 20 along the second direction "y" and also a reflection receiver adapted to generate an image of the skin surface 20 when the forearm 21 is rested on the base 2. In the embodiment of figure 2, two lateral first and second cameras 4, 5 are provided, being disposed at different lateral opposite positions in the structure of the device 1. In a preferred embodiment, the position of both cameras 4, 5 is fixed. In this fixed position, the lateral cameras have an angle of vision that are capable of see almost the entire forearm, one on one side and the other on the other side. The light beam generator 3 is moveable in a transverse direction "x" to the second direction "y", see figure 1 to illuminate another part of the forearm 21 of the patient. "x" direction corresponds to the first direction.

The general operation of the device 1 is as follows: once the patient has been put in contact with the allergic substances and the stipulated time has elapsed for them to react, he places a part of his body, generally its forearm 21, inside the device 1's structure with the anterior side of the forearm 21 facing the transverse head part 6, see figure 1. In this position, the area having papules (P1, P2) of the forearm 21 may be correctly scanned by the device 1. This scanning of all the area of interest of the forearm lasts only between 5 and 10 seconds. Once the scanning operation is complete, the patient can now withdraw the arm from the device 1. Meanwhile, the software of the computer system can detect the papules of the patient and displays the information of each papule found on a screen.

As the area of the forearm 21 of a patient to be scanned is not so long in "x" direction (corresponds to the direction transverse to the longitudinal direction of the forearm 21) -taking into account that the laser generator is moved in "x" direction"-, the two-dimensional sensor of the at least one camera 4 may quickly capture an image of the skin surface 20 of the forearm 21 area and generate a three-dimensional representation of the area of interest of the forearm 21.

Such a 3d representation of the area of interest of the forearm 21 of a patient may be formed by a cloud of points, wherein these points or a selection of these points are nodes of a mesh generated by a triangulation process performed on the data obtained from the received reflection. Once an image is captured, the computer system (22) determines one point of the cloud per pixel illuminated responsive to the intrinsic parameters of the camera; that is, the position and orientation of the sensor and the optical elements of the camera including the optical axis and the location of the optical focus. When two images are simultaneously captured, each point of the cloud is determined from the two images wherein now each image uses a pixel corresponding to the same point illuminated by the laser beam and, imposing that the determined cloud point has a minimum distance to the two lines, each line connecting the corresponding focus of the camera and the corresponding pixel.

In some preferred examples, the light beam emitted onto the forearm 21 may be a line. Such a line may be formed with a suitable lens system. The 3d representation may thus be obtained by combining a plurality of sections of the forearm 21 obtained by the line projection. In those examples, the image generated by one 4 or two lateral cameras 4, 5 comprising a curve caused by the light beam 3a when the skin surface 20 is operatively illuminated by the light beam generator 3.

According to an embodiment the system 1 may optionally include a movable unit (not shown in the figures) for displacing the light beam generator 3 to another position to emit light on another part of the area to be scanned of the forearm 21. Once this movable unit is displaced along certain position in axis "x", a plurality of line beams 3a are captured by the reflection cameras 4, 5, thus generating a plurality of two-dimensional sections of the area of the forearm 21 being scanned. At the end of the scanning process, the plurality of two-dimensional sections may be used to generate a 3d representation 8 of an area of the skin surface 20 of a forearm 21 of the person. According to a preferred embodiment, the system 1 uses a light beam generator 3 fixed to the transverse head part 6 with an optical element allowing to emit a laser beam sweeping a line according to a plain that can change the tilt to scan the entire area.

In particular reference to figure 3, a perspective view of a photograph of a part of the exterior skin surface 20 of a forearm 21 of a patient is shown, once the "Prick Test" is performed on the surface skin 20 of a forearm 21 of a patient for detecting allergies. As is it appreciated, three different papules (P) in different positions on the skin surface that project from the skin surface in the form of a protuberance. Each of the papules (P) have a different shape and dimensions.

In particular reference to figure 4, the same photograph as previous figure 3 is shown, but in this case a certain plane PL1 positioned in space x-y-z is represented generating a cross-section of the surface of the skin shown in the photograph. This plane PL1 or section contains the reflected images captured by the at least one camera of the line projection emitted by the light beam. The reflected images captured by the at least one camera forms a curve C1 positioned in space x-y-z (see next figure 5 where said curve C1 in plane PL1 is represented).

In particular reference to figure 6, the same photograph as figures 3 and 4 is shown, but in this case not only one plane PL1 but three different planes PL1₁, PL1₂, PL1₃ are represented cross-sectioning the surface of the skin. Each plane PL1₁, PL1₂, PL1₃ contains the respective reflected image captured by the at least one 4 or two cameras 4, 5 according to a first curve C1₁, C1₂, C1₃positioned in space x-y-z. These different planes PL1₁, PL1₂, PL1₃can be either parallel between them or having another configuration, such as the pages of an open book. From the different first curves C1₁, C1₂, C1₃ of a plurality of planes PL1₁, PL1₂, PL1₃ of the forearm 21 obtained by the line projection positioned in space a 3d representation 8 of a generated numerical model of an area of the skin surface 21 of a person is generated in the computer system (see figure 7).

In the following figures 7 to 12 different steps which take place in the computer system are represented.

In particular reference to figure 7, a 3d representation 8 of a numerical model generated by a computer system of an area of a skin surface 20 of a patient (i.e. the reconstruction surface) according to this example is shown. In this case, a skin surface 20 of a 3d reconstruction of an area of a forearm 21 of a patient is represented. This figure 7 also includes, four different numerical curved lines C2, which has been previously identified as "first curved lines", extended on the surface of the 3d representation 8 of the numerical model of the area of the skin surface 20 generated in a computer system. The projection of each first curved line C2 on a predetermined domain Ω is oriented according to a predetermined first direction.

In particular reference to figure 8, the step "a" of the method is represented. This figure is a graphic showing a specific vertical cut of a 3d representation 8 of a generated numerical model of an area of the skin surface 20 of a forearm 21 of a patient containing one single papule P1 is represented. In this graphic the horizontal axis is the pixels and the vertical one is the height (mmm). This vertical cut of a 3d representation 8 is constituted by a first curved line C2 and the position of the single papule P1 is highlighted by a square in the graph.

In particular reference to figure 9, the step "b" of the method for the same example of a 3d representation 8 as figure 8 is represented. In particular, this graph shows the gradient of the specific second curved line of the vertical cut of figure 8 being calculated in the computer system. In this graphic the horizontal axis is the pixels and the vertical one is the gradient (mm/px). From this graph, the computer system finds those regions (called "RS" region) of the gradient wave containing a positive wave ("R" wave) followed by a negative wave ("S" wave) of this curve. One possible search method is by searching pairs of local maximum and minimum peaks that follow pattern of behaviour of a "RS" wave region. One possible way to find the pairs of local maximum and minimum peaks that follow pattern of behaviour of a "RS" wave region is individually analyzing each pair of local maximum and minimum peaks to see if they comply with the following conditions:
- That the ratio between width (difference between maximum and minimum peak in pixels) and height (difference in height of the peaks) is not higher than a limit. In this case, all pairs except the selected one do not comply with this ratio.
- Additionally, pairs of maximum and minimum peaks that have a width below or above a threshold are filtered out.

Each of the detected "RS" regions corresponds to a starting limit point and an end limit point of an area corresponding to a protuberance that can be a papule or vein that will be next eliminated to achieve a correct "base surface". In this graph only one protuberance being a papule P1 or a vein to be after eliminated is detected and is highlighted in a rectangle.

In particular reference to figure 10, another step of the method for the same example of a 3d representation 8 as figures 8 and 9 is represented. In this graph, the original signal (i.e. a second curved line of the 3d representation of a generated numerical model of an area of the skin surface of a forearm of a patient) from which the part corresponding to a papule (i.e. a detected RS" peaks regions in step "b") has been eliminated, so managing to eliminate papules and/or veins, etc. from the original signal. In this graphic the horizontal axis is the pixels and the vertical one is the height (mm).

In particular reference to figure 11 shows step "e" of the method, where an interpolation is performed to obtain a "clean" "base surface" of the skin surface of a forearm of a patient. This figure 11 depicts a cut interpolated signal, which would represent a "clean" base of the skin surface of a forearm of a patient. In this graphic the horizontal axis is the pixels and the vertical one is the height (mm).

In particular reference to figure 12 shows step "f" of the method, where a final new signal, after subtracting the original signal of the 3d representation of said one or more papules from the base surface curve of previous step "e", is depicted. This final new signal is the one used for detecting the papules P1, P2 and also measuring the at least one dimension of the detected papules P1, P2. In this case, only a single papule P1 is detected. In this graphic the horizontal axis is the pixels and the vertical one is the height (mm).

In particular reference to figure 13, it shows a graphic where a specific vertical cut (of step "a") of a 3d representation of a generated numerical model of an area of the skin surface of a forearm of a patient containing seven different papules. In the case of figure 13 this vertical cut generates a first curved line C2. The position of the papules P1, P2 to P7 in this first curved line C2 is highlighted by circles. In this graphic the horizontal axis is the pixels and the vertical one is the height (mm).

In particular reference to figure 13, it shows a graphic where the gradient of the specific first curved line C2 of the vertical cut of figure 13 is calculated in the computer system and represented in the graphic. The computer system also searches the "RS" peaks regions corresponding to those papules or veins that will be after eliminated to achieve a correct base surface. These "RS" peaks regions are represented by a rectangle in this graph as P1, P2 to P7.

## Claims

1. Computer-implemented method of detecting and optionally measuring at least one dimension of one or more protuberances, preferably one or more papules (P1, P2), of an area of a skin surface (20) of a person or part of it, by means of a computer system (22); the method comprising the steps:
a) receive in the computer system (22) at least a three-dimensional representation (8) of a numerical model of an area scanned from the skin surface (20) of a person and determine in the computer system (22) a plurality of numerical first curved lines (C2) extended on the surface of the three-dimensional representation (8) of the numerical model of an area of the skin surface (20), wherein the projection of each first curved line (C2) on a predetermined domain (Ω) is oriented according to a predetermined first direction;
b) determine in the computer system (22), for each first curved line (C2), a starting limit point and an end limit point of an area corresponding to a protuberance, preferably a papule (P1, P2), where the starting limit point and an end limit point correspond to a local maximum followed by a local minimum or, a local minimum followed by a local maximum respectively of the gradient along the second direction;
c) determine in the computer system (22) the perimeter border of each protuberance, preferably a papule (P1, P2), in the three-dimensional representation (8) by connecting the detected starting limit points and end limit points of an area corresponding to a protuberance, preferably a papule (P1), of adjacent first curved lines (C2₁, C2₂); and
d) determine in the computer system (22) at least one dimension of at least one protuberance, preferably at least one papule, responsive to the shape of the perimeter of said protuberance, preferably said papule.

2. A method according to claim 1, wherein it further comprises the steps:
e) add in the numerical model by means of the computer system (22) an inner area delimited within each identified perimeter border of a protuberance, preferably a papule (P1, P2), from the three-dimensional representation (8) by reconstructing it by interpolation from an adjacent region of the three-dimensional representation (8) containing the protuberance, preferably the papule (P1); and
f) determine in the computer system (22) the space (9) limited between the three-dimensional representation of said one or more protuberances, preferably one or more papules (P1, P2), and the inner interpolated area intended for reconstructing the surface with no protuberances, being the at least one dimension of one or more protuberances, preferably one or more papules (P1, P2), determined from the shape of said limited space (9).

3. A method according to claim 1 or 2, by means of:
- a base (2) intended for supporting the person or part of the person, the skin surface (20) extended at least along a second direction when in operative manner;
- a light beam generator (3) configured to emit a light beam to illuminate a line projected onto the skin surface (20) along the second direction, and
- a first camera (4) comprising a first sensor, the first camera being adapted to capture the projected line onto the skin surface (20) and determinate its shape by generating an image of the skin surface (20) when the person or part of it is rested on the base (2) and; wherein the generated image also comprises a curve caused by the light beam when the skin surface (20) is operatively illuminated by the light beam generator (3), the first camera (4) at least comprising two optical parameters, the orientation of the focal axis and the location of the focal point; and
- a computer system (22) in communication with the light beam generator (3) and the first camera (4);
the method, before step a), further comprises the steps 1), 2) and 3):
step 1) capture a plurality of images of an area of the skin surface (20) of the person or part of it when, in operative manner, the person or part of it is resting on the base (2), the plurality of images taken by the first camera (4) while illuminating the area of the skin surface (20) by the light beam generator (3) and, each image taken while illuminating a line at a different location of the area of the skin surface (20);
step 2) receive in the computer system from the first camera (4) the plurality of images and determine a plurality of numerical second curved lines in a space, at least one curved line for each image of the plurality of images, each second curved line in space determined by calculating the spatial position of a set of pixels of the illuminated line appearing in the image and responsive to the optical parameters of the first camera (4); and
step 3) generate in the computer system (22) a numerical model comprising a three-dimensional representation (8) of an area of the skin surface (20), the model determined from the plurality of second curved lines in space.

4. A method according to any previous claim, wherein the light beam is a laser beam or a LED beam.

5. A method according to any claims 3 or 4, wherein the person or part of it is resting along the second direction and the focal axis of the first camera (4) is transverse to the second direction.

6. A method according to any previous claim, wherein the predetermined first direction is the second direction.

7. A method according to any of claims 3 to 7, wherein the predetermined domain is parallel to the base (2) and the projection of the first curved lines in the predetermined domain are straight parallel lines and, being equispaced.

8. A method according to any previous claim, wherein a pair of starting limit point and end limit point is discarded if they are separated in the first direction more than a first predetermined length value.

9. A method according to any previous claims, wherein a pair of starting limit point and end limit point is discarded if they are separated in a direction transversal to the second direction more than a second predetermined length value.

10. A method according to any claim 3 to 9, further comprising:
- a second camera (5) comprising a second sensor (5.1), wherein the second camera (5) is adapted to generate an image of the skin surface (20) of a person or part of it when is rested on the base (2) and the curve caused by the light beam when operatively illuminated by the light beam generator (3), the second camera (5) at least comprising two optical parameters, the orientation of the focal axis and the location of the focal point;
- a synchronization module between the first (4) and the second camera (5) to obtain pairs of images at the same instant of time; and
the computer system being in communication with the light beam generator (3), the first (4) and second (5) cameras and the synchronization module, wherein in step 1) the computer system is further configured to:
i. command the activation of the light beam generator (3) illuminating the skin surface (20) with the light beam (3a) capturing a first image with the first camera (4) and a second image with the second camera (5) along a plurality of sections of the skin surface (20) extended at least along the first direction, each pair of the first image and the second image comprising pixels corresponding to the same illuminated line on the skin;
ii. for each pair of the first image and the second image, determining a plurality of points in the space satisfying that each point corresponds to the intersection of a first straight line connecting an illuminated pixel of the first image and the location of the focal point of the first camera (4) and, a second straight line connecting an illuminated pixel of the second image of the same location on the skin surface (20) and the location of the focal point of the second camera (5); and
iii. generating the curved line of step 2) by connecting the set of points determined in former step ii).

11. A method according to any claim 3 to 10, wherein further a lens distortion of the first camera (4), a lens distortion of the second camera (5) or both are corrected by the computer system after capturing the image.

12. A method according to any claims 3 to 11, wherein the computer system (22) is further adapted to execute a sub-step of applying a first filtering stage applied to the three-dimensional representation (8) after being determined from the plurality of second curved lines in space or applied to the 2D images, wherein said first filtering stage is configured to filter out any irregularities of the three-dimensional representation (8) or to the 2D images below a first predetermined length scale, thereby generating a first pre-filtered three-dimensional representation to be used in step 2)*.*

13. A method according to previous claim, wherein the computer system (22) is further adapted to execute a sub-step of applying (11) a second filtering stage to the first pre-filtered three-dimensional representation, wherein said second filtering stage is configured to further filter out any irregularities of the first pre-filtered three-dimensional representation below a second predetermined size and over the first predetermined length scale, so generating a second pre-filtered three-dimensional representation to be used in step 2).

14. A detecting and optionally measuring system (10) of at least one dimension of one or more protuberances, preferably one or more papules (P1, P2), of an area of a skin surface (20) of a person or part of it, the system (10) comprising:
- a base (2) intended for supporting the person or part of it, the skin surface (20) extended at least along in a second direction when the system is in operative manner;
- a light beam generator (3) configured to emit a light beam (3a) to illuminate a line projected onto the skin surface (20) along the second direction, and
- a first camera (4) comprising a first sensor adapted to generate an image of the skin surface (20) when the person or part of it is rested on the base (2) and, the image also comprising a curve caused by the light beam when the skin surface (20) is operatively illuminated by the light beam generator (3), the first camera (4) at least comprising two optical parameters, the orientation of the focal axis and the location of the focal point;
- a computer system (22) in communication with the light beam generator (3) and the first camera (4), the computer system (22) adapted to:
command the first camera (4) causing to capture a plurality of images of an area of a skin surface (20) of a person or part of it resting on the base (2), the plurality of images taken by the first camera (4) while illuminating the area of the skin surface (20) by the light beam generator (3) and, each image taken while illuminating a line at a different location of the area of the skin surface (20);
receive at least from the first camera (4) the plurality of images and to determine a plurality of numerical second curved lines in a space, at least one curved line for each image of the plurality of images, each second curved line in space determined by calculating the spatial position of a set of pixels of the illuminated line appearing in the image and responsive to the optical parameters of the first camera (4), in particular the focal axis and the location of the focal point;
generate a numerical model comprising a three-dimensional representation (8) of an area of the skin surface (20), the model determined from the plurality of second curved lines in space;
determine a plurality of numerical first curved lines (C2) extended on the surface of the three-dimensional representation (8) of the numerical model, wherein the projection of each first curved line (C2) on a predetermined domain (Ω) is oriented according to a predetermined first direction;
determine, for each second curved line (C2), a starting limit point and an end limit point of an area corresponding to a protuberance preferably a papule (P), where the starting limit point and an end limit point correspond to a local maximum followed by a local minimum or, a local minimum followed by a local maximum respectively of the gradient along the first direction;
determine the perimeter border of each protuberance, preferably a papule (P), in the three-dimensional representation (8) by connecting starting limit points and end limit points of adjacent first curved lines;
determine at least one dimension of at least one protuberance, preferably at least one papule, responsive to the shape of the perimeter said protuberance, preferably said papule;
making the at least one dimension available for instance by means of an output communication line or a display.

15. A system (10) according to claim 14, wherein the computer system (22) is further adapted to:
add in the numerical model an inner area delimited within each identified perimeter border of a protuberance, preferably a papule (P), from the three-dimensional representation (8) by reconstructing it by interpolation from an adjacent region of the three-dimensional representation (8) containing the protuberance preferably a papule (P1); and
determine the space (9) limited between the three-dimensional representation (8) of said one or more protuberances, preferably one or more papules (P1, P2), and the inner interpolated area intended for reconstructing the surface with no papules, being the at least one dimension of one or more protuberances, preferably one or more papules (P1, P2), determined from the shape of said limited space (9).

16. A system (10) according to claim 14 or 15, further comprising:
- a second camera (5) comprising a second sensor, wherein the second camera (5) is adapted to generate an image of the skin surface (20) of a person or part of it when is rested on the base (2) and, the curve caused by the light beam (3a) when operatively illuminated by the light beam generator (3), the second camera (5) at least being **characterized by** two optical parameters, the orientation of the focal axis and the location of the focal point;
- a synchronization module between the first camera (4) and the second camera (5) configured to obtain pairs of images at the same instant of time; and
the computer system being in communication with the light beam generator (3), the first (4) and second cameras (5) and the synchronization module, wherein the computer system (22) is further configured to:
i. command the activation of the light beam generator (3) illuminating the skin surface (20) with the light beam (3a), an sending a signal to the first (4) and second camera (5) capturing a first image with the first camera and a second image with the second camera along a plurality of sections of the skin surface (20) extended along the first direction, each pair of the first image and the second image comprising pixels corresponding to the same illuminated line on the skin surface (20);
ii. for each pair of the first image and the second image, determining a plurality of points in the space satisfying that each point corresponds to the intersection of a first straight line connecting an illuminated pixel of the first image and the location of the focal point of the first camera (4) and, a second straight line connecting an illuminated pixel of the second image of the same location on the skin surface (20) and the location of the focal point of the second camera (5); and
iii. generate the curved line (C1) by connecting the set of points determined in former step ii).

17. A system (10)) according to any one of claims 14 to 16, wherein any one of the cameras (4, 5) are monochrome cameras.

18. A system (10) according to any one of claims 16 to 17, wherein the first (4) and second cameras (5) are located in a respective fixed position at respective lateral positions in respect to the position intended for the skin surface (20) and wherein the first (4) and second cameras (5) are angled at 90º to each other.

19. A system (10) according to any one of claims 14 to 18, wherein the light beam generator (3) is configured to project a light beam (3a) parallel to the first direction and the light beam generator (3) is displaceable along a first direction, perpendicular to the second direction and parallel to the base (2).

20. A computing device comprising a non-transitory memory and a processor, wherein the memory stores computer program instructions executable by the processor, said instructions comprising functionality to execute method of detecting at least one dimension of one or more protuberances, preferably one or more papules (P1, P2), of an area of a skin surface (20) of a person or part of it according to steps a) to e) and optionally steps 1), 2) and 3) of any of claims 1 to 13.

21. A computer program product comprising instructions which, when the program is executed by a computer system (22), cause the computer system to perform the steps a) to d) and optionally additional steps d) to f) and optionally steps 1), 2) and 3) of any of claims 1 to 13.
